# FASCICULE DE BREVET EUROPEEN

(11) **EP 0 315 499 B1**
(45) Date de publication et mention de la délivrance du brevet: **07.04.1993**
(21) Numéro de dépôt: 88402530.5
(22) Date de dépôt: 06.10.1988
(51) Int. Cl.: C07H 19/06, C07H 21/00

(54) **Dérivés de nucléosides, notamment de désoxy-2' cytidine et leur utilisation pour la synthèse d'oligonucléotides**
Nukleosid-Derivate, insbesondere von 2'-Deoxycytidin, und ihre Anwendung in der Synthese von Oligonukleotiden
Nucleoside derivatives, particularly of 2'-deoxycytidine, and their use in the synthesis of oligonucleotides

(30) Priorité: 08.10.1987 FR 8713911
(43) Date de publication de la demande: 10.05.1989
(73) Titulaire: COMMISSARIAT A L'ENERGIE ATOMIQUE, 75015 Paris Cédex 15 (FR)
(72) Inventeur: Molko, Didier, F-38210 Tullins-Fures (FR); Roget, André, F-38600 Fontaine (FR); Schulhof, Jean-Claude, F-38330 Saint Ismier (FR); Téoule, Robert, F-38000 Grenoble (FR)
(74) Mandataire: Mongrédien, André

(56) Documents cités:
- EP-A- 0 090 789
- TETRAHEDRON LETTERS, vol. 23, no. 26, 1982, pages 2615-2618, Pergamon Press Ltd, Oxford, GB; K.K. OGILVIE et al.: "N-levulination of nucleosides"
- TETRAHEDRON, vol. 40, no. 1, 1984, pages 87-94, Pergamon Press Ltd, Oxford, GB; T.G. HECKLER et al.: "Preparation of 2'(3')-O-Acyl-pCpA derivatives as substrates for T4 RNA ligase-mediated "Chemical Aminoacylation""

## Description

La présente invention a pour objet de nouveaux dérivés de nucléosides et leur utilisation pour la synthèse d'oligonucléotides.

De façon plus précise, elle concerne des dérivés de nucléosides utilisables en particulier pour la synthèse d'oligonucléotides, et parmi ceux-ci, ceux qui sont formés à partir de cytosine, c'est-à-dire d'une base pyrimidique comportant un groupement NH₂ exocyclique.

La synthèse d'oligonucléotides consiste à lier entre eux des nucléosides par un groupement phosphate pour former une chaîne d'ADN (acide désoxyribonucléique) ou d'ARN (acide ribonucléique). Dans cet assemblage, les groupements phosphates internucléotidiques relient toujours la fonction hydroxyle en position 3′ d'un nucléoside à la fonction hydroxyle en position 5′ d'un autre nucléoside. Ainsi, lors de la réaction de synthèse, seules les extrémités 3′ et 5′ des nucléosides sont sollicitées et la base nucléique (purique ou pyrimidique) utilisée ne doit pas intervenir au cours de cet assemblage.

Lorsque ces bases comprennent des groupements NH₂ exocycliques, il est nécessaire de protéger ces groupements lors de la synthèse des oligonucléotides car ceux-ci sont trop réactifs et risquent d'interférer avec les réactions de synthèse.

Cette protection des groupements NH₂ exocycliques doit répondre à certaines caractéristiques :
- elle doit être sélective et facile à réaliser,
- elle ne doit pas induire de modifications de réactivité des autres sites du nucléoside et être stable pendant toutes les étapes de la synthèse oligonucléotidique, et
- elle doit pouvoir être éliminée ensuite dans des conditions douces sans détruire l'oligonucléotide qui vient d'être synthétisé.

Les groupements NH₂ exocycliques des nucléosides ont le plus souvent été protégés sous la forme d'amides, par exemple au moyen de groupes benzoyle ou anisoyle dans le cas de l'adénine et de la cytosine comme il est décrit par H. SCHALLER et al dans J. Amer. Chem. Soc. (1963), vol. 85, p. 3821-3827, et au moyen du groupe isobutyryle dans le cas de la guanine comme il est décrit par H.BUCHI et H. KHORANA dans J. Mol. Biol. (1972), vol. 72, p. 251-288.

Le document EP-A- 090 789 décrit lui aussi la préparation de dérivés de nucléosides dans lesquels le groupement NH₂ exocyclique de la cytosine est protégé par le groupe benzoyle.

Ces groupes protecteurs peuvent être éliminés en fin de synthèse par action de l'ammoniaque à 28%, pendant 17 h, à une température de 60°C, comme il est préconisé. Cependant la RMN du proton montre que dans ces conditions on n'élimine pas tous tes groupements isobutyryle de la guanine ; il est donc préférable de porter te temps de réaction à 72 heures, toujours à une température de 60°C.

Ce mode d'élimination des groupes protecteurs constitue un inconvénient car les conditions mises en oeuvre ne sont pas suffisamment douces pour pouvoir être utilisées avec des nucléosides modifiés, peu stables en milieu alcalin, comme c'est le cas par exemple de la dihydro-5,6 thymidine.

Aussi, des recherches ont été entreprises sur la possibilité d'utiliser d'autres groupements acyle plus faciles à éliminer, utilisables en particulier pour la synthèse d'oligonucléotides à partir de nucléosides labiles par la méthodologie de synthèse sur support. Celle-ci consiste à fixer le premier nucléoside de la chaîne sur un support, généralement en silice, et à effectuer ensuite successivement des cycles de condensation pour fixer sur le premier nucléoside les autres nucléosides dans l'ordre voulu. L'utilisation de groupements acyle plus faciles à éliminer permet par ailleurs d'obtenir un meilleur rendement de déprotection. Ce point est très important car la présence de bases incomplètement déprotégées présente un inconvénient pour l'utilisation des produits obtenus.

Au cours de ces recherches, on a trouvé que les groupements NH₂ exocycliques de nucléosides formés à partir de bases pyrimidique (cytosine) ou puriques (adénine, guanine) pouvaient être protégés par un groupe acyle de formule : 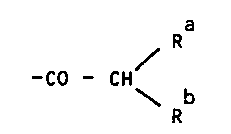
dans laquelle R^{a} représente un atome d'hydrogène ou un radical alkyle et R^{b} représente un atome d'hydrogène, un radical alkyle, un radical alcoxy ou un radical aryloxy éventuellement substitué. Ce radical peut être notamment le groupe phénoxyacétyle pour les nucléosides formés à partir de la guanine et de l'adénine, et le groupe isobutyryle pour les nucléosides formés à partir de la cytosine. En utilisant ces nouveaux radicaux protecteurs, les conditions opératoires de déprotection peuvent être adoucies de façon significative puisque l'on peut obtenir une déprotection complète par traitement à l'ammoniaque pendant deux heures à la température ambiante.

Cependant, certains problèmes spécifiques sont encore présents pour les nucléosides formés à partir de la cytosine car la N-4-isobutyryl désoxy-2′ cytidine a une stabilité trop importante par rapport à celle des dérivés (N6-phénoxyacétyl)-désoxy-2′ adénosine et du dérivé (N2-phénoxyacétyl)-désoxy-2′ guanosine. En effet, 30 min sont nécessaires pour obtenir la demi-déprotection du dérivé de cytidine alors que 15 min suffisent pour le dérivé de désoxy-2′ guanosine et 7 min seulement pour le dérivé de désoxy-2′ adénosine.

Par ailleurs, on a rencontré certaines difficultés dans la préparation du dérivé de N4-isobutyryl désoxy-2′ cytidine. En effet, ce dérivé est préparé par réaction du chlorure d'isobutyryle avec la désoxy-2′ cytidine, mais on obtient dans ces conditions un dérivé triprotégé du nucléoside de départ, c'est-à-dire un dérivé dans lequel le groupe isobutyryle protège non seulement les groupes NH₂ exocycliques, mais les fonctions hydroxyles du groupement désoxyribose. Aussi, on doit hydrolyser ce dérivé triprotégé, puis séparer du milieu réactionnel et des nombreux sels qu'il contient le dérivé monoprotégé obtenu après hydrolyse. Ceci peut être réalisé par une extraction à l'aide d'un solvant organique approprié mais cette étape manque de reproductibilité, sans doute à cause des solubilités respectives dans l'eau de l'acide isobutyrique et du dérivé recherché. Les impuretés qui restent présentes dans la phase aqueuse gênent alors la purification du dérivé de nucléoside protégé, par cristallisation.

Aussi, les recherches ont été poursuivies pour trouver d'autres radicaux protecteurs du groupement NH₂ exocyclique de la désoxy-2′ cytidine permettant d'éviter ces différents inconvénients.

La présente invention a précisément pour objet de nouveaux dérivés de nucléosides formés à partir de la cytosine, qui comportent des groupements protecteurs plus faciles à éliminer que le groupe isobutyrique et qui sont par ailleurs plus faciles à préparer avec un degré de pureté satisfaisant.

Ces dérivés de nucléosides répondent à la formule : 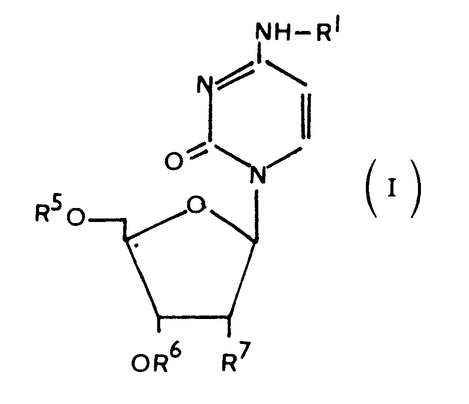
dans laquelle R¹ représente le radical de formule :

-CO-CH₂-C(R²R³)-R⁴

dans laquelle R² et R³ qui peuvent être identiques ou différents, représentent un atome d'hydrogène ou un radical alkyle, et R⁴ représente un radical aryle non substitué ou substitué par un ou plusieurs groupements choisis parmi :
NO₂ , CN, alcoxy, aryloxy, Cl, F, 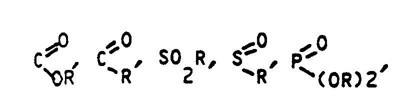
alkyle et SR avec R représentant un radical alkyle ou aryle,
R⁵ représente un atome d'hydrogène, un radical labile en milieu acide ou le radical de formule R¹,
R⁶ représente un atome d'hydrogène, un radical phosphoré ou le radical R¹, et
R⁷ représente un atome d'hydrogène ou le radical OH(protégé ou non).

L'utilisation selon l'invention d'un radical R¹ répondant à la formule précitée permet de diminuer le temps nécessaire pour déprotéger le dérivé de nucléoside du radical R¹ après son utilisation pour la synthèse d'oligonucléotides. Par ailleurs, avec ce radical R¹, on peut obtenir le dérivé monoprotégé de la désoxy-2′ cytidine dans de bonnes conditions, avec un bon degré de pureté et un rendement supérieur à 70%.

En effet, dans ce radical R¹ la présence des substituants R², R³ et R⁴ permet d'augmenter la différence de solubilité entre le nucléoside protégé sur le groupement NH₂ exocyclique et l'acide carbonique correspondant au radical R¹. On peut ainsi extraire plus facilement l'acide carboxylique par une phase éthérée alors que le nucléoside protégé demeure suffisamment polaire pour se maintenir dans une phase aqueuse.

Par ailleurs, la présence des substituants R², R³ et R⁴ en position béta de la fonction carbonyle correspond à une distance entre le centre actif et la substitution suffisamment grande pour limiter l'augmentation de la vitesse d'hydrolyse et éviter ainsi que le dérivé de nucléoside ne soit trop instable. En effet, lorsqu'ils sont protégés par les mêmes radicaux protecteurs, les dérivés de la désoxy-2′ cytidine se déprotègent 20 à 50 fois plus rapidement que leurs homologues puriniques. Dans l'invention, on choisit les substituants et leur positionnement pour diminuer la durée de déprotection du nucléoside et la rendre comparable à celles qui sont nécessaires pour déprotéger les autres dérivés de nucléosides tels que la N6-(phénoxyacétyl) désoxy-2' adénosine.

Ainsi, l'invention permet d'obtenir rapidement le dérivé protégé avec un rendement supérieur à 70%, et le temps de demi-déprotection du dérivé de nucléoside de l'invention, dans les conditions standard, peut être de 8 min, c'est-à-dire une valeur proche de celle que l'on obtient dans les mêmes conditions pour la N6-(phénoxyacétyl) désoxy-2' adénosine (7 min) et la N2-phénoxyacétyl désoxy-2' guanosine (15 min). Il s'agit donc d'une nette amélioration par rapport à la valeur de 30 min qui était nécessaire auparavant avec la N4-isobutyryl désoxy-2' cytidine.

Dans le radical R¹ du dérivé de nucléoside de l'invention, R² et R³ peuvent représenter un atome d'hydrogène ou un radical alkyle et R⁴ est un radical aryle éventuellement substitué.

Les radicaux alkyle susceptibles d'être utilisés peuvent être linéaires ou ramifiés, ils ont généralement de 1 à 4 atomes de carbone.

Le radical aryle utilisé est en particulier le radical phényle, mais on peut utiliser tout autre radical dérivé d'un noyau par élimination d'un atome d'hydrogène, par exemple les radicaux naphtyle, anthracényle,... Ce radical aryle peut comporter un ou plusieurs substituants choisis parni NO₂, CN, alcoxy, aryloxy, Cl, F, C(O)OR, C(O)R, SO₂R, S(O)R, PO₃R₂, alkyle et SR avec R représentant un radical alkyle ou aryle. Les radicaux alkyle utilisés comme substituants peuvent être du même type que R² et R³. Les radicaux alcoxy utilisables comme substituants, ont généralement de 1 à 4 atomes de carbone et peuvent être linéaires ou ramifiés.

Les radicaux aryle utilisés comme substituants peuvent être les radicaux phényle, naphtyle ou anthracényle.

De préférence, dans l'invention, R¹ et R² représentent un atome d'hydrogène et R⁴ représente le radical phényle.

Selon un exemple de réalisation de l'invention, R⁵ et R⁶ représentent un atome d'hydrogène.

Selon un autre exemple de réalisation de l'invention, R¹ représente le radical -CO-CH₂-CH₂-C₆H₅, R⁵ représente : 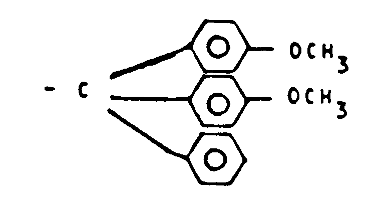
R⁶ représente le radical 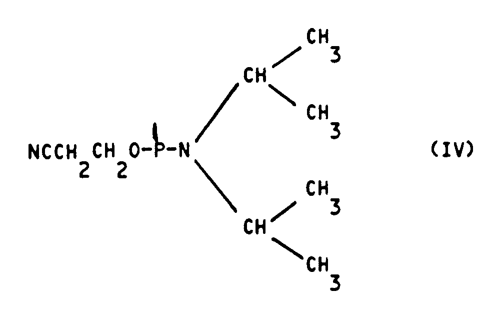
et R⁷ représente un atome d'hydrogène.

Dans les dérivés de nucléosides de l'invention, les radicaux labiles en milieu acide susceptibles d'être utilisés pour former R⁵ sont en particulier des radicaux utilisables en synthèse oligonucléotidique tels que :
- les radicaux trityle répondant à la formule : 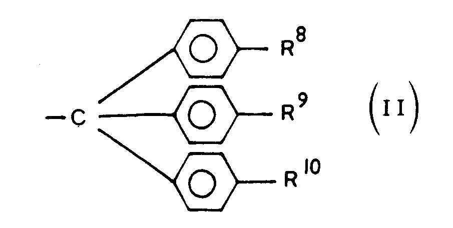 dans laquelle R⁸, R⁹ et R¹⁰ qui peuvent être identiques ou différents, représentent un atome d'hydrogène, un radical alkyle ou un radical alkoxy, par exemple le radical monométhoxytrityle ou le radical trityle de formule (V) dans laquelle R⁸ et R⁹ représentent le radical méthoxy et R¹⁰ représente un atome d'hydrogène ;
- les radicaux pixyle et les radicaux phényl-9 xanthényle.

Dans les dérivés de nucléosides de l'invention, les radicaux phosphorés susceptibles d'être utilisés pour former R⁶ dans le composé de formule (I) sont également des radicaux utilisables en synthèse oligonucléotidique tels que le radical de formule : 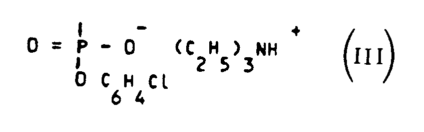
le radical de formule : 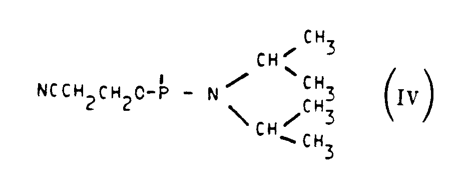
le radical phosphonate de formule : 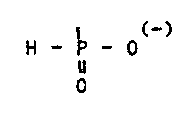
Généralement, on utilise le radical de formule (IV).

Selon l'invention, lorsque R⁷ représente le radical OH protégé, on utilise comme groupe protecteur de OH les groupes habituellement utilisés dans la synthèse des ribonucléotides.

Les dérivés de nucléosides de l'invention sont ainsi les produits de l'union 1°) d'une base formée par la cytosine et 2°) du ribose ou du désoxyribose, les nucléosides étant modifiés au moins sur le groupement NH₂ exocyclique de la cytosine par le groupement R¹ . Ils peuvent également être modifiés par ce même groupement sur les positions 3′ et 5′ du désoxyribose ou 2′, 3′ et 5′ du ribose, ou les positions 3′ et 5′ du ribose ou du désoxyribose peuvent être modifiées par d'autres groupements qui sont les groupements labiles R⁵ pour la position 5′ et le groupement phosphoré R⁶ pour la position 3′ du ribose ou du désoxyribose.

Les groupements acyle de formule :

-CO-CH₂(R¹R²)-R⁴

utilisés dans l'invention sont particulièrement intéressants pour la synthèse des oligonucléotides car ils peuvent être éliminés facilement en fin d'opération, par exemple par traitement à l'ammoniaque pendant 2 h à la température ambiante, ce qui permet de libérer simultanément l'oligonucléotide du support sur lequel il a été synthétisé, lorsqu'on utilise la méthode de synthèse sur support. Ce temps est comparable au temps qui était nécessaire avec les nucléosides protégés par le groupement :

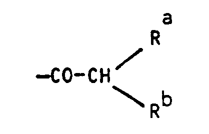

On peut également opérer dans des conditions réactionnelles douces puisqu'on réalise la déprotection à température ambiante et l'utilisation de ce groupe protecteur éliminable facilement permet d'incorporer lors de la synthèse des oligonucléotides des bases nucléiques modifiées sensibles à des conditions alcalines plus violentes, par exemple de synthétiser des fragments d'ADN porteurs de ligands sensibles à des anticorps.

Bien que l'invention s'applique aux nucléosides dérivés du ribose et aux nucléosides dérivés du désoxyribose, on l'utilise de préférence pour les nucléosides dérivés du désoxyribose, c'est-à-dire les dérivés de formule (I) dans laquelle R⁷ est un atome d'hydrogène.

Les dérivés de nucléosides de l'invention peuvent être préparés par des procédés classiques, analogues à ceux utilisés pour fixer les groupements benzoyle et anisoyle sur les nucléosides à base d'adénine ou de cytosine. Dans ces procédés, on part du nucléoside de la cytosine que l'on fait réagir avec le chlorure d'acide de formule R¹ Cl ou l'anhydride d'acide de formule :

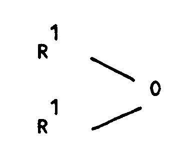

Pour éviter que lors de cette réaction, le chlorure d'acide ou l'anhydride d'acide réagisse également avec les groupements hydroxyle en position 3′ et 5′ du ribose ou du désoxyribose, on protège tout d'abord ces groupements hydroxyle en les faisant réagir avec un composé approprié, qui peut être le chlorure de triméthylsilyle, dans un solvant approprié tel que la pyridine. Après réaction du nucléoside de la cytosine avec le chlorure d'acide R¹Cl ou l'anhydride d'acide (R¹)₂O, on élimine les groupes protecteurs des groupements hydroxyle en position 3′ et 5′ du ribose ou du désoxyribose par hydrolyse, par exemple en utilisant une solution d'ammoniaque.

Selon une variante de préparation du dérivé protégé par le groupement R¹, on effectue la réaction sans protéger les groupements hydroxyle en position 3′ et 5′ du ribose ou du désoxyribose, puis on soumet à une hydrolyse sélective le dérivé de nucléoside triprotégé.

On peut préparer les dérivés de nucléosides de formule (I) dans laquelle R⁵ représente un radical trityle de formule (II), par exemple le groupement diméthoxytrityle, et R⁶ représente un atome d'hydrogène en faisant réagir les dérivés des nucléosides obtenus précédemment avec le chlorure de trityle correspondant dans un solvant approprié.

Les dérivés de nucléosides répondant à la formule (I) dans laquelle R⁵ représente un groupement diméthoxy-trityle ou méthoxy-trityle et R⁶ représente soit le radical de formule (III), soit le radical de formule (IV), soit un radical de formule: 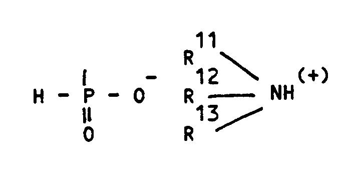
dans laquelle R¹¹, R¹² et R¹³ qui peuvent être identiques ou différents, sont des radicaux alkyle, par exemple éthyle, peuvent être préparés par des procédés classiques à partir des dérivés de nucléosides de formule(I) dans laquelle R⁵ représente un radical diméthoxy-trityle ou méthoxy-trityle et R⁶ représente un atome d'hydrogène.

Pour préparer les dérivés dans lesquels R⁶ représente, par exemple, le radical de formule (III), on fait réagir ce dérivé de nucléoside avec du bistriazolidate de chloro-4 phénylphosphoryle dans un solvant approprié. Le bistriazolidate de chloro-4 phénylphosphoryle peut être préparé par addition de dichlorophosphate de chloro-4 phényle à une suspension de triazole et de triéthylamine dans du dioxanne.

Pour préparer les dérivés dans lesquels R⁶ représente, par exemple, le radical de formule (IV), on peut faire réagir le dérivé de nucléoside avec du β-cyanoéthyl-monochloro N,N-diisopropyl amino phosphoramidite dans un solvant approprié en présence de diisopropyléthylamine ou bien avec du β cyanoéthylbis (N,N diisopropylamino) phosphite en présence de tétrazole et de diisopropylamine.

Pour préparer les dérivés de nucléosides dans lesquels R³ représente, par exemple, le radical de formule : 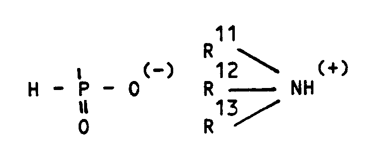
dans laquelle R¹¹, R¹² et R¹³ qui peuvent être identiques ou différents, sont des radicaux alkyle, on peut faire réagir le dérivé de nucléoside avec de la chloro-2 benzo(5,6-a) [dioxo-1,3 phosphor-2 inone-4] puis avec un sel de trialkyl ammonium tel que l'acétate de triéthyl ammonium.

Les dérivés de nucléosides obtenus par ces trois méthodes peuvent être utilisés pour la synthèse d'oligonucléotides soit par synthèse phosphotriester dans le cas où R⁶ est le radical de formule (III), soit par synthèse phosphoramidite dans le cas où R⁶ est le radical de formule (IV), soit par synthèse H-phosphonate dans le cas où R⁶ est le radical de formule : 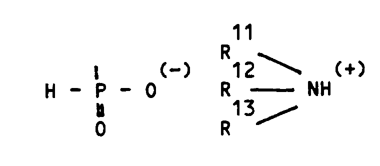
en utilisant également pour l'assemblage des chaînes oligonucléotidiques d'autres nucléosides, par exemple ceux correspondant à la thymidine et à la désoxy-2′ uridine, ou encore des nucléosides comportant des bases labiles en milieu alcalin ou d'autres nucléosides labiles en milieu alcalin.

Le procédé, selon l'invention, de synthèse d'oligonucléotides, comprend :
1° au moins un cycle de condensation dans lequel on condense sur un dérivé de nucléoside ou sur un oligonucléotide un dérivé de nucléoside de formule : 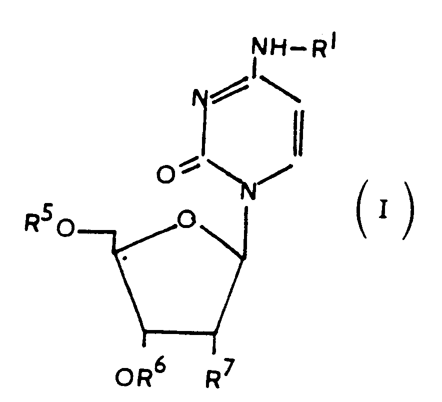 dans laquelle R¹ représente le radical de formule :

   -CO-CH₂-C(R²R³)-R⁴

   dans laquelle R² et R³ qui peuvent être identiques ou différents, représentent un atome d'hydrogène ou un radical alkyle, et R⁴ représente un radical aryle non substitué ou substitué par un ou plusieurs groupements choisis parmi :
   NO₂, CN, alcoxy, aryloxy, Cl, F, 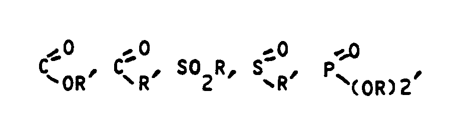 alkyle et SR avec R représentant un radical alkyle ou aryle, R⁵ représente un radical labile en milieu acide, R⁶ représente un radical phosphoré et R⁷ représente un atome d'hydrogène, et
2°) une étape d'élimination du ou des groupes protecteurs de formule R¹ .

Cette étape peut être réalisée par exemple par mise en contact de l'oligonucléotide avec de l'ammoniaque à la température ambiante.

La synthèse oligonucléotidique peut être effectuée soit par des méthodes en solution, soit par des méthodes de synthèse sur support. On utilise de préférence les méthodes de synthèse sur support car celles-ci sont mieux adaptées à l'utilisation de nucléosides plus labiles, sans qu'il y ait une perte de rendement au cours de l'assemblage.

Les nucléosides de l'invention peuvent donc trouver des applications intéressantes en tant que produits de base destinés à la synthèse de fragments d'ADN ou d'ARN. Ils peuvent également convenir pour incorporer dans des oligonucléotides de synthèse des bases modifiées fragiles, ce qui peut concerner en particulier les produits de radiolyse gamma de l'ADN ainsi que les produits de photolyse.

Les nucléosides de l'invention peuvent permettre également l'accès à de nouvelles molécules à activité antivirale et à de nouvelles sondes d'ADN.

Les exemples suivants de préparation et d'utilisation des nucléosides conformes à l'invention sont donnés bien entendu à titre non limitatif pour illustrer l'invention.

### EXEMPLE 1 : Préparation de N4-(phényl-3-propionyl,)-désoxy-2′ cytidine (composé n^{o}1)

On sèche 20 mmol de désoxycytidine puis on les dissout dans 100 ml de pyridine anhydre ; on ajoute alors à cette solution 10 ml de chlorure de triméthylsilyle (80 mmol) et on laisse la réaction évoluer à température ordinaire pendant 25 minutes. On additionne alors 14,6g (50 mmol) d'anhydride phénylpropionique et on laisse la réaction se poursuivre pendant 17 heures à 4°C.

On a alors formé un nucléoside dont les fonctions alcool en position 3′ et 5′ sont protégées par un groupement triméthylsilyle et dont la fonction amine exocyclique est sous la forme d'une amide.

On libère les fonctions hydroxyles 3′ et 5′ en additionnant 20 ml d'eau et une solution d'ammoniaque concentrée jusqu'à l'obtention d'un pH de 8. Il y a alors formation d'un précipité blanc qui est éliminé de la solution par filtration. On évapore ensuite les solvants sous pression réduite. On reprend le résidu huileux de couleur orangée obtenu par 500 ml d'eau et on lave cette phase aqueuse par un mélange d'éther éthylique et d'acétate d'éthyle dans les proportions 80%/20% (3x500 ml). On concentre alors la phase aqueuse jusqu'à apparition d'un léger trouble et on l'abandonne pendant 17 heures à 4°C. Par filtration on récupère 3,8g de N4-(phényl-3 propionyl) désoxy-2′ cytidine. On concentre à nouveau les eaux mères, on les abandonne au froid afin de recueillir une nouvelle fraction de 1,3g de produit, ce qui correspond à un rendement global de 71%.

On caractérise le produit par chromatographie sur couche mince, spectrométrie de masse et résonance magnétique nucléaire du proton à 200 MHz. Les résultats obtenus sont les suivants :
- Rf : 0,70 dans le mélange de migration chloroforme-méthanol (80/20),
- Spectrométrie de masse (M-H) : pic moléculaire (m/e=359-78%),
- Résonance magnétique nucléaire du proton à 200 MHz dans l'acétone deutériée : 8,55 ppm (d, 1H, H5), 7,44 ppm (d, 1H, H6), 6,33 ppm (t,1H, H1′), 2,57 ppm (8 raies, 1H, H2′), 2,30 ppm (6 raies, 1H, H2˝), 4,60 ppm (6 raies, 1H, H3′), 4,13 ppm (q, 1H, H4′), 3,98 ppm (q, 1H, H5′), 3,91 ppm (q, 1H, H5˝), 3,20-2,90 ppm (m, 4H, Ethyle du propionyle), 7,42-7,38 ppm (m, 5H, phényle).

### EXEMPLE 2 : Préparation de N4-(phényl-3 propionyl)(diméthoxy-4,4′-trityl)-5′désoxy-2′cytidine (composé n^{o}2)

On sèche 8 mmol du composé n^{o}1 (3g) et on le reprend dans 50 ml de pyridine anhydre. On refroidit à 0°C et on ajoute 3g (8,8 mmol) de chlorure de diméthoxy-4,4′ trityle. On laisse évoluer la réaction à 5°C pendant 17 heures et on ajoute 2 ml de méthanol. Après 30 min, on chasse le solvant à l'évaporateur rotatif et on reprend le résidu huileux par 300 ml d'acétate d'éthyle, on le lave par 150 ml d'une solution aqueuse 5% de bicarbonate de sodium et deux fois par 150 ml d'eau. On sèche alors la phase organique par du sulfate de sodium et on fractionne le mélange sur une colonne de gel de silice. On isole le composé n^{o}2 avec un rendement de 40%. Les caractéristiques physico-chimiques de ce composé sont les suivantes :
- Rf : 0,25 dans le mélange chloroforme-méthanol (90/10)=0,25,
- spectrométrie de masse (M-H) : pic moléculaire m/e=660-8%,
- résonance magnétique nucléaire du proton à 200 MHz : 8,39 ppm (d, 1H, H5), 7,70-6,90 ppm (m, 19H, H5+aromatiques), 6,31 ppm (t, 1H, H1′), 2,62 ppm (8 raies, 1H, H2′), 2,35 ppm (8 raies, 1H, H2˝), 4,68 ppm (m, 1H, H3′), 4,23 ppm (q, 1H, H′4), 3,57 ppm (d, 2H, H5′, H5˝), 3,92 ppm (s, 6H, méthoxyles).

### EXEMPLE 3 : Préparation du dérivé phosphorylé du composé n^{o}2 destiné à la synthèse phosphoramidite d'oligonucléotides (composé n^{o}3)

On sèche 1,6g du composé n°2 par addition et évaporation de 50 ml de dichlorométhane anhydre, stabilisé sur amylène et on les reprend par 50 ml de dichlorométhane. On les ajoute à un autre ballon contenant 0,170 ml de diisopropylamine, 85 mg de tétrazole et 1,05g de bis-(diisopropylamino) cyanoéthyl phosphine dans 25 ml de dichlorométhane. Après 3 heures de réaction à la température ordinaire, on lave cette phase organique par 100 ml de solution aqueuse de bicarbonate de sodium à 5% (deux fois) et 100 ml d'eau. On évapore ensuite le solvant et on fractionne le mélange sur une colonne de gel de silice éluée par le mélange ternaire suivant : chloroforme 60%, hexane 35%, triéthylamine 5%. On rassemble les fractions contenant le produit recherché pur, on évapore le solvant et on reprend le résidu huileux incolore par 15 ml de toluène. On le verse goutte à goutte dans 250 ml d'hexane préalablement refroidi à -80°C. On recueille ainsi un précipité du composé n^{o}3 (1,3g, 62%), caractérisé par les données physico-chimiques suivantes :
- Rf : deux taches correspondant aux deux diastéréoisomères 0,57 et 0,064,
- spectrométrie de masse (M+H) : pic moléculaire m/e=862-1,2%,
- résonance magnétique nucléaire à 200 MHz : le spectre complexe d'un tel mélange de produits est très difficile à dépouiller. Seuls les signaux des protons suivants ont pu être attribués avec certitude. H1′ 6,33 ppm (q), H3′ 4,85 ppm (m), H4′ 4,35 ppm (m), H5′ et H5˝ 3,60 ppm (m), H2˝ 2,49 ppm (m), H5 8,40 ppm (dd), méthoxyle 3,90 ppm (m), méthyles 1,2-1,4 ppm (m).

### EXEMPLE 4 :

Cet exemple illustre l'utilisation du composé n^{o}3 pour la synthèse d'oligonucléotides dont les séquences sont les suivantes :
- 5′ AAT CAG ATC TAC GAA TTC T 3′ (19 mère),
- 5′ ATC AGT GCA GGG ACC GAG ATG TGC TCC AAG GAG TGT TTA TCG GCT GCT T 3′ (49 mère),
- 5′ TGC AGT CGG CTT TCG TCA CGT CCC TGG GTG TAC ACG AGG TTC CTC AGA AAT AGC CGA CGA AAG TCT ATG CTT 3′ (72 mère).

Dans ces séquences, A représente le nucléotide formé avec l'adénine, T représente le nucléotide formé avec la thymine, C représente le nucléotide formé avec la cytosine, G représente le nucléotide formé avec la guanine. Pour effectuer ces synthèses, on utilise le composé n^{o}3 comme synthon correspondant à la cytosine. Les synthons correspondant à l'adénine, la guanine et la thymine sont préparés de la façon suivante.

### A) Synthon correspondant à l'adénine

Ce synthon est le dérivé phénoxyacéthylé de l'adénine qui répond à la formule suivante :

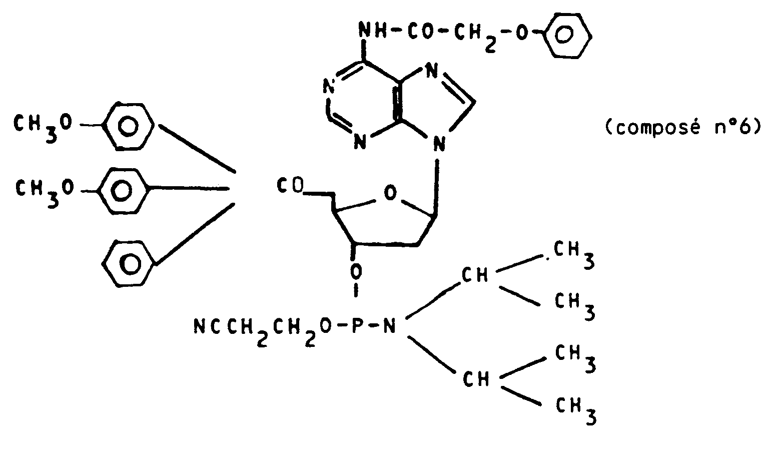

On le prépare à partir de la désoxy-2′ adénosine en effectuant les étapes suivantes :

### a) - Préparation de N6-(phénoxyacétyl)-désoxy-2′ adénosine (composé n^{o}4)

On sèche 1025 mg (4 mmol) de désoxyadénosine puis on les dissout dans 20 ml de pyridine distillée anhydre et on les introduit dans un ballon placé dans un bain d'eau glacée. On ajoute alors lentement 8 équivalents d'anhydride phénoxyacétique (9,5 g ; 32 mmol) dissous dans 20 ml de pyridine à 0°C. On laisse la réaction se poursuivre à la température ambiante pendant 90 min et une coloration jaunâtre apparaît progressivement. On forme ainsi le dérivé de nucléoside triprotégé.

On détruit alors l'excès d'anhydride d'acide à 0°C par addition de 3 ml d'eau distillée, puis on dilue le milieu réactionnel par 100 ml de chloroforme. On lave la phase chloroformique 4 fois au moyen de 50 ml d'une solution aqueuse à 5% de bicarbonate de sodium, et on évapore le solvant, ce qui conduit à l'obtention d'un résidu jaune. On dissout celui-ci dans 100 ml de pyridine et, après avoir placé la solution dans un bain d'eau glacée, on ajoute 100 ml de soude 0,2 N à 0°C pour hydrolyser sélectivement les positions 3′ et 5′ de l'adénosine en 15 min. On neutralise alors le milieu au moyen de la résine échangeuse de cations Dowex 50W-X8 sous forme pyridinium. On filtre et on rince la résine puis on évapore à sec le filtrat.

On obtient ainsi la N6-(phénoxyacétyl)-désoxy-2′ adénosine (composé n^{o}4) que l'on purifie par chromatographie sur colonne de silice (diamètre 4 cm et longueur 10 cm) en utilisant un gradient de chloroforme-méthanol (100-0-96-4). On évapore alors les fractions contenant le produit recherché et l'on obtient ainsi 1010 mg d'une poudre blanchâtre, ce qui correspond à un rendement de 65%.

On caractérise alors le produit par chromatographie sur couche mince, par résonance magnétique nucléaire du proton à 250 MHz et par spectrométrie de masse. Les résultats obtenus sont les suivants :
- R_{F} : 0,66 avec le mélange de migration chloroforme-méthanol (80/20 en volume),
- résonance magnétique nucléaire du proton à 250 MHz : ¹H-RMN (pyridine d₅) : 2,7-3,3 (m, 2H, H₂, H_{2˝}) : 4,1-4,35 (m, 2H, H₅ H_{5˝}) : 4,6 (m, H_{4′}) ; 5,25 (m, H_{3′}) ; 5,65 (s, 2H, CH₂) ; 7,0 (m, H_{1′}) ; 6,9-7,4 (m, 5H, C₆ H ₅) ; 8,75 et 9,05 (s, H₂ et H₈).
- spectrométrie de masse : (M+H) : pic moléculaire (m/e : 386-16%) ; adénine phénoxy acétylée (m/e : 270 - 66%).

### b) - préparation de N6-(phénoxyacétyl)-diméthoxy-4,4′ trityl)-5′ désoxy-2′ adénosine (composé n^{o}5)

On sèche 2,5 mmol du composé n^{o} 4 par additions et évaporations successives de pyridine anhydre. On reprend par 25 ml de pyridine, on refroidit à 0°C et on ajoute 2,75 mmol (1,1 équivalent) de chlorure de diméthoxy-4,4' trityle dans 25 ml de pyridine, à 0°C. On laisse la réaction se poursuivre pendant 17 h à 5°C et on ajoute alors 2 ml de méthanol au milieu réactionnel. Après 30 min, on chasse le solvant à l'évaporateur rotatif et on reprend le résidu huileux par 100 ml d'acétate d'éthyle, et on lave par 3 fois 50 ml de solution aqueuse à 5% de NaHCO₃ et une fois 50 ml d'eau bidistillée. On sèche alors la phase organique sur sulfate de sodium et on la concentre. Par fractionnements sur colonne de gel de silice, on isole le composé n^{o}5.

### c) - Préparation du dérivé phosphorylé destiné à la synthèse phosphoramide d'oligonucléotides (composé n^{o}6).

On sèche 3 mmol du composé n^{o}5 par co-évaporation de pyridine, de toluène et de tétrahydrofuranne (THF). On reprend le résidu dans 15 ml de THF en présence de 12 mmol de N,N,N-diisopropyléthylamine et on ajoute goutte à goutte en 2 min 6 mmol de -cyanoéthyl-monochloro N,N-diisopropylaminophosphoramidite. Après 5 min de réaction, on observe la formation d'un précipité de chlorhydrate de l'amine. On laisse la réaction se poursuivre pendant 35 min et on filtre le précipité en fin de réaction, puis on évapore à sec le filtrat et on le reprend dans 150 ml d'acétate d'éthyle. On lave par une solution aqueuse glacée à 10% de Na₂CO₃. On sèche ensuite la phase organique sur sulfate de sodium et on l'évapore à sec.

On purifie le composé obtenu par chromatographie basse pression sur une colonne MERCK "LOBAR" de taille B en utilisant pour l'élution un mélange CH₂Cl₂ -hexane-triéthylamin (70/20/10 en volume). On reprend le composé obtenu par un minimum de dichlorométhane ou d'acétate d'éthyle et on le précipite dans l'hexane à -80°C.

On obtient ainsi le synthon correspondant à l'adénine (composé n^{o}6).

### B) - Synthon correspondant à la quanine

Ce synthon est le dérivé phénoxyacétylé de formule :

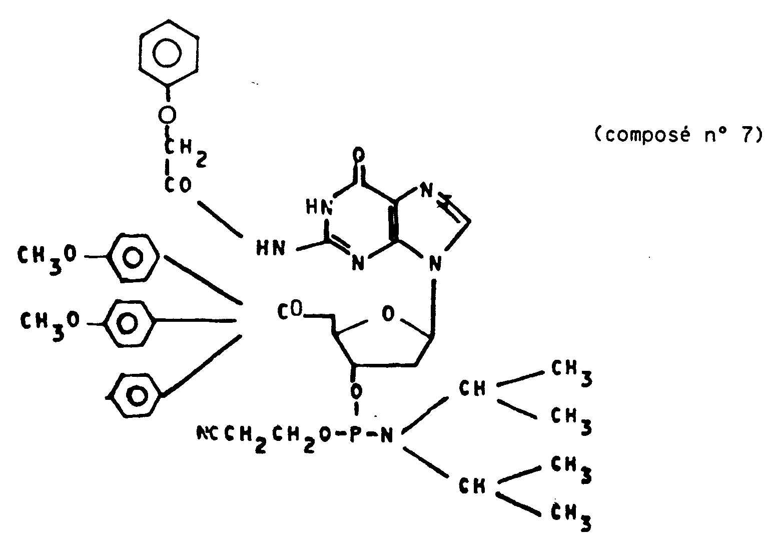

On le prépare à partir de la désoxy-2′ guanosine en utilisant le même mode opératoire que pour la préparation du synthon correspondant à l'adénine.

### C) - Synthon correspondant à la thymine

Celui-ci est obtenu en suivant le même mode opératoire que précédemment à partir de la thymidine et sans effectuer l'étape a).

### D) - Synthèse des oligonucléotides

On effectue ces synthèses au moyen de l'appareil Applied Biosystems ABS 380 A en suivant les protocoles prescrits par le fournisseur et en utilisant les réactifs chimiques provenant de ce fournisseur et les quantités suivantes de synthons :
- synthon de l'adénine (composé n^{o}6) : 400 mg dissous dans 4,48ml d'acétonitrile anhydre,
- synthon de la guanine (composé n^{o}7) : 400mg dissous dans 4,64ml d'acétonitrile anhydre,
- synthon de la cytosine (composé n^{o}3) : 400 mg dissous dans 4,72ml d'acétonitrile anhydre,
- synthon de la thymine : 400 mg dissous dans 5,28 ml d'acétonitrile anhydre.

On installe sur l'appareil des cartouches de support greffé avec la thymidine (0,2 micro mole) et on assemble les séquences d'oligonucléotides en effectuant successivement des cycles de condensation selon le protocole prescrit par le fournisseur.

En fin d'opération, on réalise la déprotection des oligonucléotides en soumettant la cartouche contenant l'oligonucléotide à l'action de l'ammoniaque concentrée, pendant 2 heures à la température ambiante. Ce traitement décroche également l'oligonucléotide, soit le fragment d'ADN, du support. On évapore alors la phase liquide à sec et on dessale le mélange de produits sur une colonne de gel d'exclusion.

Après marquage radioactif au phosphore 32 au moyen de la T4-polynucléotidekinase, on vérifie par électrophorèse sur gel de polyacrylamide que les fragments d'ADN synthétisés ont la longueur voulue.

## Revendications

1. Dérivé de nucléoside répondant à la formule: 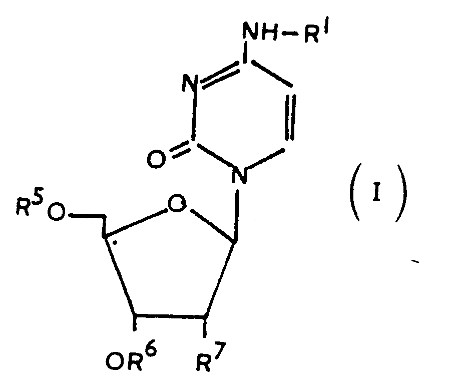 dans laquelle R¹ représente le radical de formule :
-CO-CH₂-C(R²R³)-R⁴
dans laquelle R² et R³ qui peuvent être identiques ou différents, représentent un atome d'hydrogène ou un radical alkyle, et R⁴ représente un radical aryle non substitué ou substitué par un ou plusieurs groupements choisis parmi :
NO₂, CN, alcoxy, aryloxy, Cl, F, 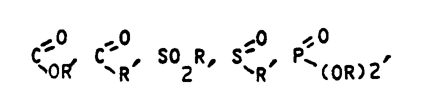 alkyle et SR avec R représentant un radical alkyle ou aryle,
R⁵ représente un atome d'hydrogène, un radical labile en milieu acide ou le radical de formule R¹ ,
R⁶ représente un atome d'hydrogène, un radical phosphoré ou le radical R¹ , et
R⁷ représente un atome d'hydrogène ou le radical OH (protégé ou non).

2. Dérivé de nucléoside selon la revendication 1, caractérisé en ce que R¹ représente le radical -CO-CH₂-CH₂-C₆ H₅.

3. Dérivé de nucléoside selon l'une quelconque des revendications 1 et 2, caractérisé en ce que le radical labile en milieu acide R⁵ est choisi dans le groupe
- des radicaux trityle de formule : 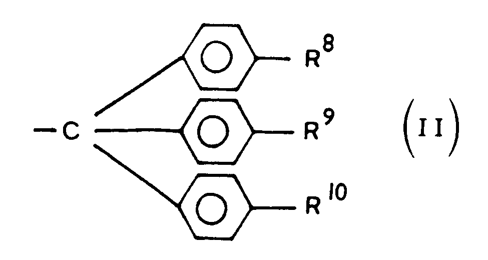 dans laquelle R⁸ , R⁹ et R¹⁰ qui peuvent être identiques ou différents, représentent un atome d'hydrogène, un radical alkyle ou un radical alcoxy,
- des radicaux pixyle et des radicaux phényl-9 xanthényle.

4. Dérivé de nucléoside selon l'une quelconque des revendications 1 à 3, caractérisé en ce que le radical phosphoré R⁶ est choisi dans le groupe des radicaux de formule : 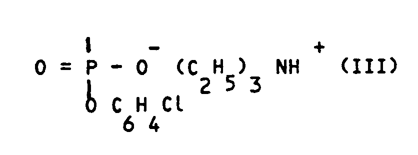 de formule : 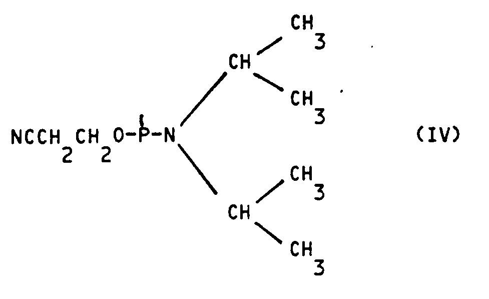 ou de formule :

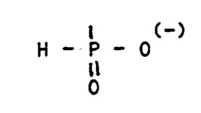

5. Dérivé de nucléoside selon l'une quelconque des revendications 1 et 2, caractérisé en ce que R⁵ et R⁶ représentent un atome d'hydrogène.

6. Dérivé de nucléoside selon l'une quelconque des revendications 1 et 2, caractérisé en ce que R⁵ représente le radical : 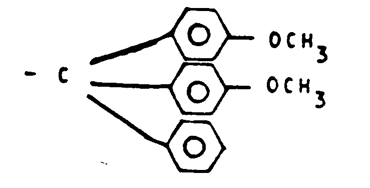 et R⁶ représente un atome d'hydrogène.

7. Dérivé de nucléoside selon l'une quelconque des revendications 1 à 6, caractérisé en ce que R⁷ représente un atome d'hydrogène.

8. Dérivé de nucléoside selon la revendication 2, caractérisé en ce que R⁵ représente le radical : 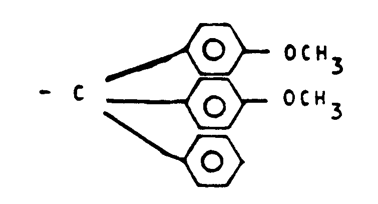 et R⁶ représente le radical :

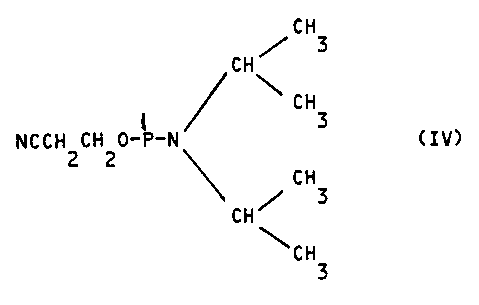

9. Dérivé de nucléoside selon la revendication 8, caractérisé en ce que R⁷ représente un atome d'hydrogène.

10. Procédé de synthèse d'oligonucléotides, caractérisé en ce qu'il comprend :
1°) au moins un cycle de condensation dans lequel on condense sur un dérivé de nucléoside ou sur un oligonucléotide un dérivé de nucléoside de formule : 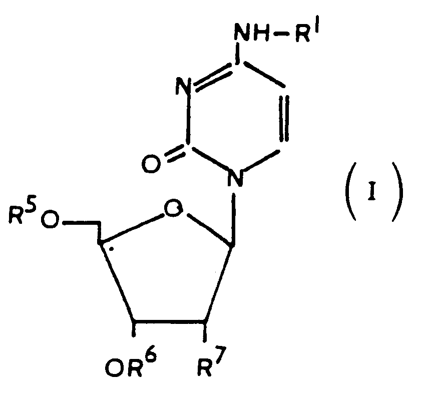 dans laquelle R¹ représente le radical de formule :
-CO-CH₂-C(R²R³)-R⁴
dans laquelle R² et R³ qui peuvent être identiques ou différents représentent un atome d'hydrogène ou un radical alkyle, et R⁴ représente un radical aryle non substitué ou substitué par un ou plusieurs groupements choisis parmi :
NO₂, CN, alcoxy, aryloxy, Cl, F, 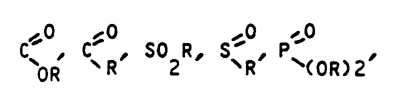 alkyle et SR avec R représentant un radical alkyle ou aryle,
R⁵ représente un radical labile en milieu acide,
R⁶représente un radical phosphoré et
R⁷ représente un atome d'hydrogène, et
2°) une étape d'élimination du ou des groupes protecteurs de formule R¹.

11. Procédé selon la revendication 10, caractérisé en ce que R¹ est le radical -CO-CH₂-CH₂-C₆H₅.

## Claims

1. Nucleoside derivative in accordance with formula: 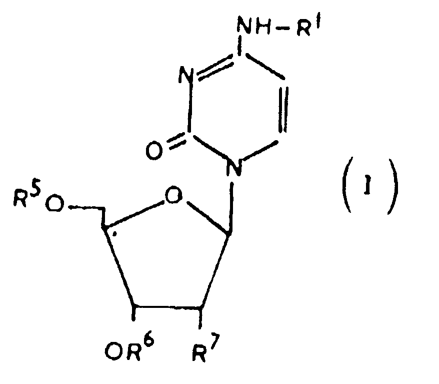 in which R¹ represents the radical of formula:
-CO-CH₂-C(R²R³)-R⁴
in which R² and R³, which can be the same or different, represent a hydrogen atom or an alkyl radical and R⁴ represents an aryl radical, which is unsubstituted or which is substituted by one or more groups chosen from among:
NO₂, CN, alkoxy, aryloxy, Cl, F, 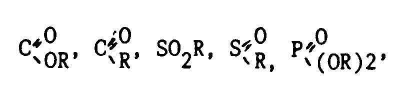 alkyl and SR with R representing an alkyl or aryl radical, R⁵ represents a hydrogen atom, a radical unstable in an acid medium or the radical of formula R¹, R⁶ represents a hydrogen atom, a phosphorus radical or the radical R¹ and R⁷ represents a hydrogen atom or the OH radical (protected or unprotected).

2. Nucleoside derivative according to claim 1, characterized in that R¹ represents the radical -CO-CH₂-CH₂-C₆C₅.

3. Nucleoside derivative according to either of the claims 1 and 2, characterized in that the radical unstable in the acid medium R⁵ is chosen from the group of trityl radicals of formula: 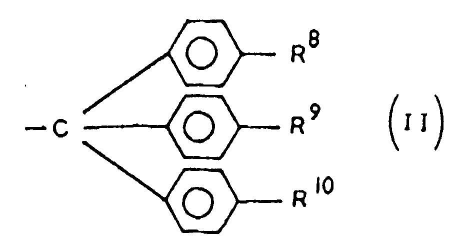 in which R⁸, R⁹ and R¹⁰, which can be the same or different, represent a hydrogen atom, an alkyl radical or an alkoxy radical, pixyl radicals and 9-phenyl xanthenyl radicals.

4. Nucleoside derivative according to any one of the claims 1 to 3, characterized in that the phosphorus radical R⁶ is chosen from the group of radicals of formula: 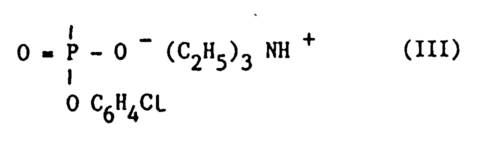 of formula: 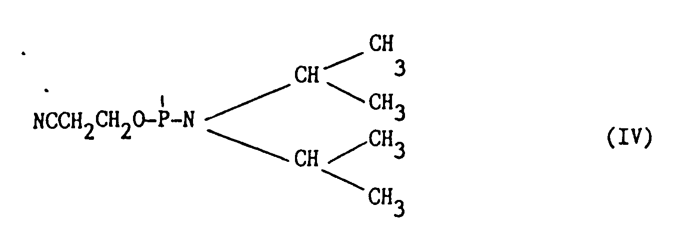 or of formula:

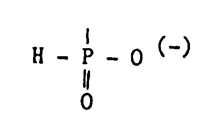

5. Nucleoside derivative according to either of the claims 1 and 2, characterized in that R⁵ and R⁶ represent a hydrogen atom.

6. Nucleoside derivative according to either of the claims 1 and 2, characterized in that R⁵ represents the radical: 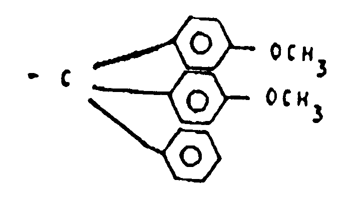 and R⁶ represents a hydrogen atom.

7. Nucleoside derivative according to any one of the claims 1 to 6, characterized in that R⁷ represents a hydrogen atom.

8. Nucleoside derivative according to claim 2, characterized in that R⁵ represents the radical: 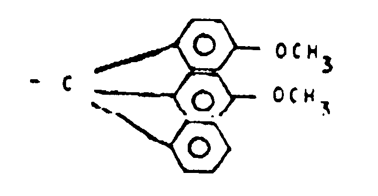 and R⁶ represents the radical:

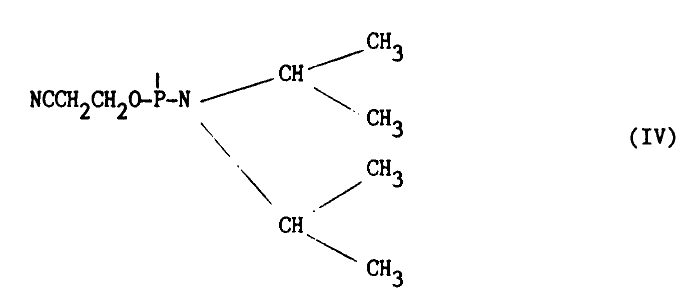

9. Nucleoside derivative according to claim 8, characterized in that R⁷ represents a hydrogen atom.

10. Process for synthesis of oligonucleotides, characterized in that it comprises:
1) at least one condensation cycle, in which on a nucleoside derivative or on an oligonucleotide is condensed a nucleoside derivative of formula: 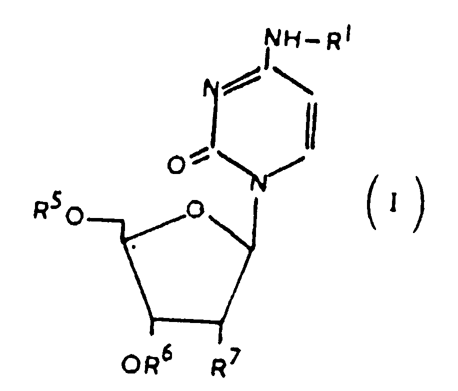 in which R¹ represents the radical of formula:
-CO-CH₂-C(R²R³)-R⁴
in which R² and R³, which can be the same or different, represent a hydrogen atom or an alkyl radical, and R⁴ represents an aryl radical, which is not substituted or is substituted by one or more groups chosen from among:
NO₂, CN, alkoxy, aryloxy, Cl, F, 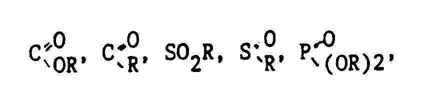 alkyl and SR with R representing an alkyl or aryl radical,
R⁵ represents a radical unstable in the acid medium,
R⁶ represents a phosphorus radical and
R⁷ represents a hydrogen atom and
2) a stage of eliminating the protective group or groups of formula R¹.

11. Process according to claim 10, characterized in that R¹ is the radical -CO-CH₂-CH₂-C₆H₅.

## Patentansprüche

1. Nukleosidderivat entsprechend der Formel: 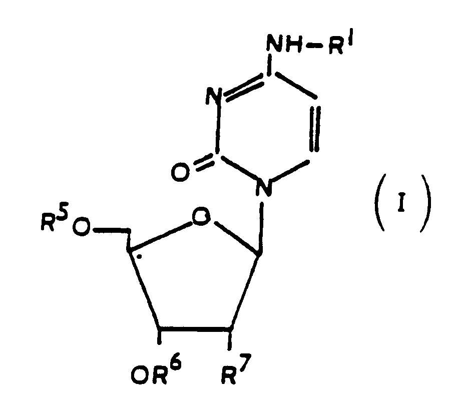 in welcher R¹ den Rest der Formel:
-CO-CH₂-C(R²R³)-R⁴
bedeutet, in welcher R² und R³, die identisch oder verschieden voneinander sein können, ein Wasserstoffatom oder einen Alkylrest bedeuten und R⁴ einen Arylrest, der unsubstituiert oder durch ein oder mehrere Gruppen, ausgewählt unter:
NO₂, CN, Alkoxy, Aryloxy, Cl, F, 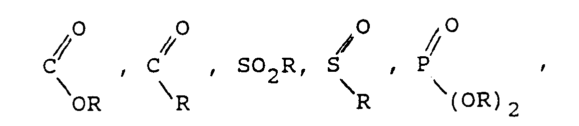 Alkyl und SR, wobei R einen Alkylrest oder Arylrest bedeutet, substituiert ist, bedeutet,
R⁵ ein Wasserstoffatom, einen in saurem Milieu labilen Rest oder den Rest der Formel R¹ bedeutet,
R⁶ ein Wasserstoffatom, einen phosphorhaltigen Rest oder den Rest R¹ bedeutet, und
R⁷ ein Wasserstoffatom oder den OH-Rest (geschützt oder nicht) bedeutet.

2. Nukleosidderivat nach Anspruch 1, **dadurch gekennzeichnet,** daß R¹ den Rest -CO-CH₂-CH₂-C₆H₅ bedeutet.

3. Nukleosidderivat nach einem der Ansprüche 1 und 2, **dadurch gekennzeichnet,** daß der in saurer Umgebung labile Rest R⁵ ausgewählt wird aus der Gruppe
- der Tritylreste der Formel: 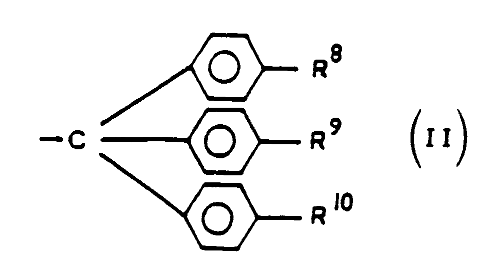 in welcher R⁸, R⁹ und R¹⁰, die identisch oder voneinander verschieden sein können, ein Wasserstoffatom, einen Alkylrest oder einen Alkoxyrest bedeuten,
- der 9-Phenyl-xanthenyl-Reste.

4. Nukleosidderivat nach einem der Ansprüche 1 bis 3, **dadurch gekennzeichnet,** daß der phosphorhaltige Rest R⁶ ausgewählt wird aus der Gruppe der Reste der Formel: 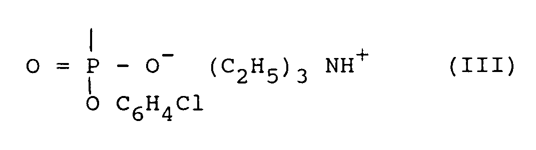 der Formel: 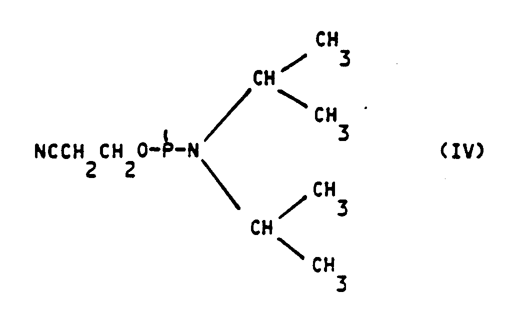 oder der Formel:

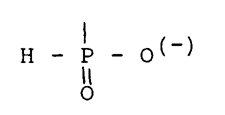

5. Nukleosidderivat nach einem der Ansprüche 1 und 2, **dadurch gekennzeichnet,** daß R⁵ und R⁶ ein Wasserstoffatom bedeuten.

6. Nukleosidderivat nach einem der Ansprüche 1 und 2, **dadurch gekennzeichnet,** daß R⁵ den Rest 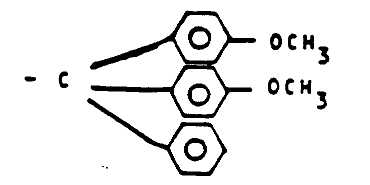 bedeutet und R⁶ ein Wasserstoffatom bedeutet.

7. Nukleosidderivat nach einem der Ansprüche 1 bis 6, **dadurch gekennzeichnet,** daß R⁷ ein Wasserstoffatom bedeutet.

8. Nukleosidderivat nach Anspruch 2, **dadurch gekennzeichnet,** daß R⁵ den Rest: 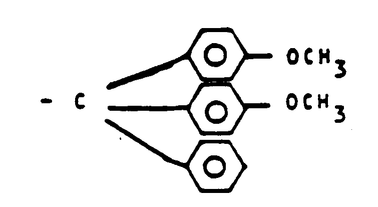 bedeutet und R⁶ den Rest: 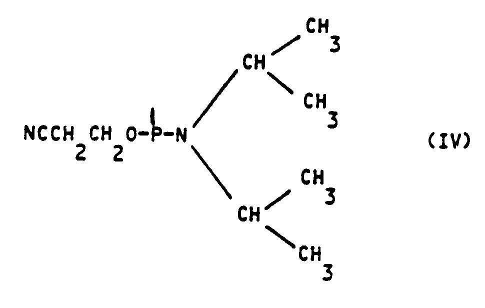 bedeutet.

9. Nukleosidderivat nach Anspruch 8, **dadurch gekennzeichnet,** daß R⁷ ein Wasserstoffatom bedeutet.

10. Verfahren zur Synthese von Oligonukleotiden, **dadurch gekennzeichnet,** daß es umfaßt:
1) mindestens einen Kondensationszyklus, in welchem mit einem Nukleosidderivat oder einem Oligonukleotid ein Nukleosidderivat der Formel: 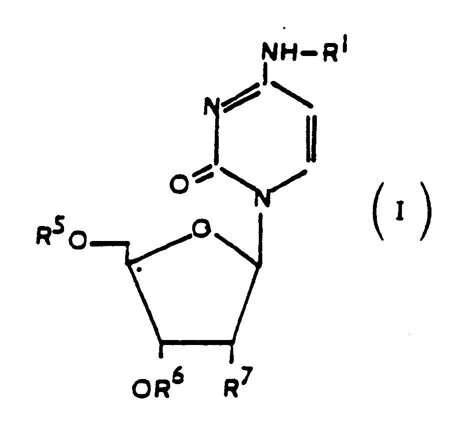 in welcher R¹ den Rest der Formel:
-CO-CH₂-C(R²R³)-R⁴
bedeutet, in welcher R² und R³, die identisch oder verschieden sein können, ein Wasserstoffatom oder einen Alkylrest bedeuten und R⁴ einen Arylrest bedeutet, der unsubstituiert oder mit einer oder mehreren Gruppen, ausgewählt aus:
NO₂, CN, Alkoxy, Aryloxy, Cl, F, 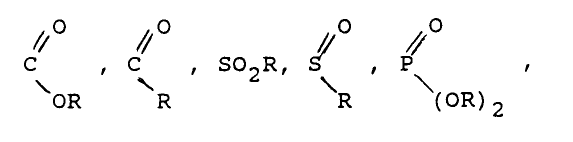 Alkyl und SR, wobei R einen Alkylrest oder Arylrest bedeutet, substituiert ist,
R⁵ einen in saurer Umgebung labilen Rest bedeutet,
R⁶ einen phosphorhaltigen Rest bedeutet, und
R⁷ ein Wasserstoffatom bedeutet, kondensiert wird, und
2) einen Schritt der Entfernung der Schutzgruppe oder der Schutzgruppen der Formel R¹.

11. Verfahren nach Anspruch 10, **dadurch gekennzeichnet,** daß R¹ der Rest -CO-CH₂-CH₂-C₆H₅ ist.
